# EUROPEAN PATENT SPECIFICATION

(11) **EP 0 342 658 B1**
(45) Date of publication and mention of the grant of the patent: **11.05.1994**
(21) Application number: 89108907.0
(22) Date of filing: 18.05.1989
(51) Int. Cl.: C12N 15/31, C12P 21/00, C12N 1/21

(54) **Biosynthetic process for the preparation of chemical compounds**
Biosynthetisches Verfahren zur Herstellung von chemischen Verbindungen
Procédé biosynthétique de préparation de produits chimiques

(30) Priority: 18.05.1988 GB 8811761
(43) Date of publication of application: 23.11.1989
(73) Proprietor: Dr. Karl Thomae GmbH, D-88397 Biberach (DE)
(72) Inventor: Jung, Günther, Prof. Dr., D-7400 Tübingen (DE); Kellner, Roland,, D-6148 Heppnheim (DE); Entain, Karl-Dieter, Prof.Dr., D-7406 Mössingen (DE); Schnell, Norbert, D-8630 Coburg (DE); Kaletta, Cortina, D-7403 Entringen (DE); Götz, Friedrich, Prof.-Dr., D-7400 Tübingen (DE); Werner, Rolf G., Dr., D-7950 Biberach (DE); Allgaier, Hermann, Dr., D-7950 Biberach (DE)

(56) References cited:
- EP-A- 342 486
- US-B- 4 716 115
- CHEMICAL ABSTRACTS, vol. 104, no. 1, 6th January 1986, page 206, abstract no.2246z, Columbus, Ohio, US; T. SHIBA et al.: "Lanthionine peptide"
- CHEMICAL ABSTRACTS, vol. 109, no. 17, 24th October 1988, page 288, abstrac no.144800c, Columbus, Ohio, US; N. SCHNELL et al.: "Prepeptide sequence of epidermin, a ribosomally synthesized antibiotic with four sulfide-rings"
- CHEMICAL ABSTRACTS, vol. 110, 2nd January 1989, page 583, abstract no. 210845v,Columbus, Ohio, US; H.P. FIEDLER et al.: "Purification of the hydrophilic antibiotics epidermin, ballidermin, and nikkomycin Z by preparative reversed-phase HPLC"
- CHEMICAL ABSTRACTS, vol. 110, no. 1, 2nd January 1989, page 395, abstract no.4243u, Columbus, Ohio, US; R. KELLNER et al.: "Gallidermin: a new lanthionine-containing polypeptide antibiotic"

## Description

This invention relates to the biosynthesis of chemical compounds, and in particular to the biosynthesis of chemical compounds containing dehydroamino acid residues and/or thioether bridges.

Some polypeptide antibiotics such as nisin, subtilin, duramycin, cinnamycin, ancovenin, Ro 09-0198 and epidermin contain dehydroamino acids and lanthionine bridges.

These polypeptides are produced by various respective strains of microorganism. Nisin for examples can be produced by cultivating strains of Streptococcus lactin, and subtilin by cultivation of Bacillus subtilis.

US Patent 4,716,115 discloses the production of nisin in Streptococcus strains by transferring recipient strains with plasmids from other, nisin-producing, Streptococcus strains.

The genetic basis for the biosynthesis of these antibiotics has not, hitherto, been elucidated. Thus, it has not been known, for example, whether biosynthesis of such antibiotics, and in particular the formation of the unusual amino acids found therein occurs via ribosomal synthesis or via multi-enzyme complexes.

In addition it was not known whether the precursor proteins of such antibiotics were coded by distinct structural genes or were the degradation products of larger proteins.

In the course of work carried out to establish the structural gene of epidermin we have been able to establish that surprisingly the above mentioned antibiotics, in particular epidermin are each coded by a distinct structural gene, and that processing of a presequence polypeptide is carried out by an enzymatic complex which effects formation of dehydroamino residues and/or thio ether bridges.

Furthermore, the multi-enzyme complex may be involved in the secretion of the protein through the cell membrane into the culture supernatant, as well as processing a prepolypeptide. In this connection, such activity may be associated with a pre-sequence possessed by the pre-polypeptide e.g. as in the case of the -30 to -1 sequence of pre-epidermin as described below.

Broadly speaking the present invention provides in one aspect a bacterial host containing a plasmid, wherein said plasmid codes for a polypeptide which is not normally produced by said host, and wherein said host during cultivation provides a multi-enzyme complex whereby a polypeptide is produced which contains at least one dehydroamino acid and/or at least one lanthionine bridge, said produced polypeptide being foreign to said host.

A suitable multi enzyme complex is one which is capable of effecting at least one of the following operations, namely water elimination and sulphide bridge formation; the complex may also effect decarboxylation and double bond formation.

Suitable hosts for carrying out the process of the present invention are those which, without modification of their genetic material, are capable of producing polypeptides containing a dehydroamino acid residue and/or a lanthionine bridge and/or a methyl lanthionine bridge. Examples of such hosts Streptococcus lactis, Bacillus subtilis, Streptomyces cinnamoneus, Streptomyces sp. Streptoverticullum griseoverticillum, Staphylococcus epidermis, Staphylococcus epidermin strain 5 and Staphylococcus gallinarum.

Strains which are of especial interest are Staphylococcus gallinarum (F16/P57) Tü 3928 which has been deposited with the Deutsche Sammlung von Microorganismen under the terms of the Budapest Treaty on 18 May 1988 and has received the depository number **DSM 4616**, and Staphylococcus epidermis **DSM 3095** which was deposited by the present applicants with the Deutsche Sammlung von Microorganismen under the terms of the Budapest Treaty on 26th October 1984 and mutant strains thereof, e.g. a mutant strain of S.epidermis DSM 3095 which is incapable of producing epidermin.

In order to transform a suitable host a suitable plasmid may be modified by known genetic engineering techniques.

Desirably a plasmid from a host which produces a polypeptide containing at least one dehydroamino acid residue and/or at least one sulfide bridge is treated by modifying or replacing the gene coding for a pre-polypeptide to provide a plasmid coding for a polypeptide foreign to said host and then transforming said host with the altered plasmid.

Any of a variety of methods may be used to replace or modify a gene coding for the pre-polypeptide.

DNA coding for the pre-polypeptide sequence of the desired compound can be prepared by chemical synthesis. Suitable chemical syntheses have been disclosed in Anal. Biochem. 121, 365 (1982). The known techniques allow the preparation of polynucleotides of up to 60 e.g. to 100 bases to be prepared.

Suitable protected nucleotides can be linked by the phosphodiester method Agarwal et al., (Angew. Chem. 84, 489 (1972)), the phosphotriester method (Reesem., Tetrahedron 39, 3, (1983)) or the phosphitetriester method (Letsinger et al., J. Am. Chem. Soc. 98, 3655 (1976)) or the phosphoramidite method. The solid phase method allows for simplification of the synthesis of the polynucleotides.

The double stranded DNA can be constructed enzymatically from chemically prepared short but overlapping segments.

For example, overlapping polynucleotide sequences from both DNA strands can be used, which are held together in the correct conformation by base pairing and are then chemically linked by the enzyme DNA ligase (Khorana et al., J. Biol. Chem. 251, 565 (1976)).

Another possibility comprises incubating in each case one polynucleotide sequence from the two DNA stands with a short overlapping segment in the presence of the four required deoxynucleoside triphosphates with a DNA-polymerase, for example DNA-polymerase I, the Klenow fragment of polymerase I or T4 DNA-polymerase, or with reverse transcriptase. The two polynucleotide sequences are thereby held together in the correct arrangement by base pairing and are supplemented with the required nucleotides by the enzyme to give a complete double-strand DNA (Narany et al., Anal. Biochem. 121, 365 (1982)).

Another suitable method for obtaining the DNA coding for a polypeptide comprises isolating the said DNA from the genomic DNA of a tissue or cell culture or microorganism, lysing the cells e.g. with SDS or proteinase K, or if desired mechanically, and deproteinising the DNA by repeated extraction with phenol.

The RNA can be preferably digested with RNase. The obtained raw DNA is partially digested with suitable restriction enzymes e.g. HaeIII and AluI and fragments isolated and multiplied in a suitable phage or cosmid, e.g. in charon 4A or EMBL-3 phage and assayed for the desired sequences e.g. with a radioactively labelled DNA probe.

The DNA coding for a desired polypeptide can also be obtained by reverse transcription of isolated mRNA into cDNA. This may be the preferred method if the DNA structure is not known. In this method the DNA is obtained from genomic DNA in a cDNA library via the mRNA. The cDNA library comprises the genetic information which is complementary to the mRNA isolated from cells.

To obtain a cDNA library the mRNA is isolated from cells expressing the desired basic (possibly unmodified) protein. This mRNA is converted into double stranded cDNA.

Standard methods well known in the art are applied in the preparation of mRNA. The cell membrane is broken and the cell content released from which the mRNA is isolated. The cell membrane is preferably broken by physical methods or lysis with detergents such as SDS, guanidine thiocyanate, definite salt conditions or homogenisation, preferably by mixing. The mRNA is isolated by the standard methods of phenol extraction, ethanol precipitation, centrifugation and chromatography, preferably a combination of several methods. Centrifugation is preferably done over gradients, for example over a CsCl gradient. For chromatography preferably columns are used, especially oligo-dT columns.

The total mRNA can be converted directly into Ds-cDNA following the methods of the art. Preferably the mRNA coding for a desired polypeptide is further enriched using several techniques, such as electrophoresis, chromatography and centrifugation, preferably sucrose gradient centrifugation.

Fractions containing mRNA coding for a desired polypeptide can be detected by various methods, such as in vivo or in vitro translations, followed by detection of a relevant activity or, when the nucleotide sequence is known, by hybridisation with an oligonucleotide probe.

In vivo translation systems can be prokaryotic or eukaryotic systems. A preferred in vivo translation system is the Xenopus laevis oocyte system (see Maniatis et al., Molecular Cloning, A Laboratory Manual, Cold Spring Harbor Laboratory (1982)). In vitro systems are for example wheat germ and rabbit reticylocyte lysates, both of which are commercially available.

From any pool of mRNA derived from unfractionated or fractionated mRNA, dS-CDNA can be obtained by the well known methods of the art (preferred general methods are described in Maniatis et al. (supra) Okayam and Berg, Molecular and Cell Biology 2, 161-170 (1982) and Heidecker, Nucleic Acid Research 11, 4891-4906 (1983)). In general the mRNA is converted first to ss-cDNA using reverse transcriptase or DNA-polymerase I (Klenow fragment). Two methods are alternatively used for priming the synthesis of the ds-cDNA. The first method was the natural loop formation of the ss-cDNA. The second method is that of tailing the ss-cDNA with a homopolymeric tail such as poly-dC or poly-DT.

The mRNA fraction of which the corresponding polypeptide shows the highest activity in the detection system is transcribed into the complementary cDNA by methods well known in the art. The mRNA and oligo-dT as a primer are mixed, dNTPs are then added as starting material and the synthesis of the cDNA-mRNA hybrid molecule is realised by the enzyme reverse transcriptase. The RNA molecules are degraded by addition of NaOH. DNA polymerase is admixed, preferably the Klenow fragment of the DNA polymerase I and the mixture is incubated at a suitable temperature preferably 12-15°C. The mixture is incubated with nuclease S1 and the ds-cDNA corresponding to the mRNA coding for a desired polypeptide is obtained.

For amplification the obtained ds-cDNA can be spliced into a suitable vector e.g. the plasmid pUC-KO and the obtained hybrid vector multiplied by use of a suitable host, e.g. E.coli HB101. Reisolation of the hybrid vectors, and recovering the isolated cDNA therefrom allows a structure determination of the DNA coding for a desired polypeptide.

### Preparation of a hybrid vector

A hybrid vector of the invention can be prepared by splicing a DNA coding for a polypeptide of the desired sequence into a suitable vector.

Suitable vectors are carriers for integrated passenger DNA, which can be used to transform a host microorganism.

Suitable as vectors are plasmids derived from microorganisms which in an untransformed state produce polypeptides which contain dehydroamino and/or sulfide groups. Suitable vectors carry the insert DNA at a defined position.

In general, such vectors may contain a replicon and a control sequence, i.e. a promoter, which are derived from the host cell or a species compatible with the host cell in which they are used. The vector ordinarly carries a replicon site and may contain sequences (marker genes) which are capable of providing phenotype selection in transformed cells. Suitable marker genes may provide antibiotic resistance or resistance to heavy metals or they may complement a genetic defect of the host. Further useful sequences in such vectors are enhancer and activator sequences.

One suitable starting vector is a 54 Kbp plasmid pEpi32 from the strain Staphylococcus epidermis DSM 3095. This plasmid, which is characterised below contains the epiA gene coding for a 52-prepeptide, which is processed to a tetracyclic 21-peptide amide antibiotic.

A vector carrying a passenger DNA is designated a hybrid vector.

The desired DNA is spliced into the starting vector by conventional methods.

A starting plasmid for example can first be linearised by a suitable restriction enzymes, e.g. the plasmid pEpi32 by HindIII, BamHI and EcoRI, then d/G-tailed in the presence of dGTP and the terminal deoxynucleotidyl transferase. The double stranded cDNA insert is dC-tailed in the presence of dCTP and terminal deoxynucleotidyl transferase. Combining both cDNA and vector results in the hybrid vector. Bacterophages, such as lamda, are preferred for constructing genomic libraries. The lambda cloning systems are described by Maniatis (supra). The suitable vector DNA is digested to completion with the appropriate restriction enzyme, and the left and right arms are separated from the central fragments by velocity gradient centrifugation or gel electrophoresis. Another method is to digest parts of the stuffer fragments with restriction enzymes which lack recognition sites in the left and the right arms. The isolated genomic DNA can be partially digested to fragments of 13-20kb in length. Afterwards the arms are ligated with the fragments of foreign DNA having termini compatible with those of the arms.

The appropriate DNA insert is recloned from the original vector used for the original cloning, into a suitable expression vector. To this end appropriate restriction enzymes are used, possibly in combination with oxonucleones, to produce the desired DNA fragments.

The DNA insert may be subcloned into a multiple site of a suitable well known plasmid vector e.g. derivatives of pC194, pTI81 and pUB110 at the restriction sites HindIII/BamHI/EcoRI.

The method of the invention can thus be used to prepare derivatives of known peptides and hormones, in which a cystein residue in the unmodified peptide is replaced by sulfide-bridged amino acids and serine and thiamine are replaced by corresponding dehydroamino acid residues.

These fragments are integrated into an appropriate expression vector by using the cohesive ends directly or by the addition of appropriate chemically synthesised oligonucleotide bridges. For the modification of the ends for example HindIII and BglII can be used. The method is not limited to any special restriction enzymes. Any desired link can be made between the expression vector and the DNA insert using suitable restriction enzymes in combination with chemically synthesised oligonucleotides.

Appropriate DNA inserts can also be obtained which code for polypeptides having site directed mutagenesis.

A variety of methods may be used to induce mutations of underlying DNA so as to prepare the desired mutants.

One method may comprise first inserting a fragment of a native or basic gene, containing sequences coding for the region to be mutated, into the replicative form of a phage e.g. phage MI3mp8 to form MI3mp8PA. A synthetic oligonucleotide, complementary to the inserted sequences but containing one or more nucleotidetriplets which code for the amino acid to be substituted, is then annealed to the single stranded form of MI3mp8A to form a double stranded region. This region serves as a primer for DNA polymerase I synthesis of the remaining complementary strand. After replication and identification, the mutant sequence may be further modified or used to construct a suitable vector for expressing the mutated polypeptide.

In the work carried out on epidermin a wobbled DNA probe 5′-GTG(A)CAT(G/A)ATG(A)AAT(C)TT-3′ deduced from a suitable pentapeptide segment of the proposed pre-sequence of epidermin. LysPheIleCylThr was prepared. This DNA probe was hybridised against plasmid DNA from S. epidermin DSM 3095.

Restriction analysis of the isolated plasmid reveals seven DNA fragments with EcoRI (16, 11, 10, 6.5, 5.5, 3.5 and 2.5 kbp), nine DNA fragments with HindIII (17, 14, 10, 5.3, 2.8, 1.8, 0.8, 0.6 and 0,5 kbp) and five DNA fragments with BamHI (20, 19, 10, 3 and 1 kbp).

A 5.4 kbp HindIII fragment was subcloned and subjected to rehybridisation whereby the structural gene epiA was located within a 2.2 kbp EcoRI/BglII fragment.

As a mixture of 24 different 14-mers was used as a hybridisation probe. The probe was applied in a 30-fold excess as a sequencing primer in accordance with the techniques described in Novick et al. Ann. N.Y. Acad. Sci. 182, 279-294 (1971), Southern, J. Molec. Biol. 98, 503-517 (1975) and Heinrich et al., Molecul. gen. Genet. 209, 563-569 (1987). The peptide sequence of epidermin allowed identification of the open reading frame. A single methionine codon is in appropriate distance to a Shine-Dalgaro sequence.

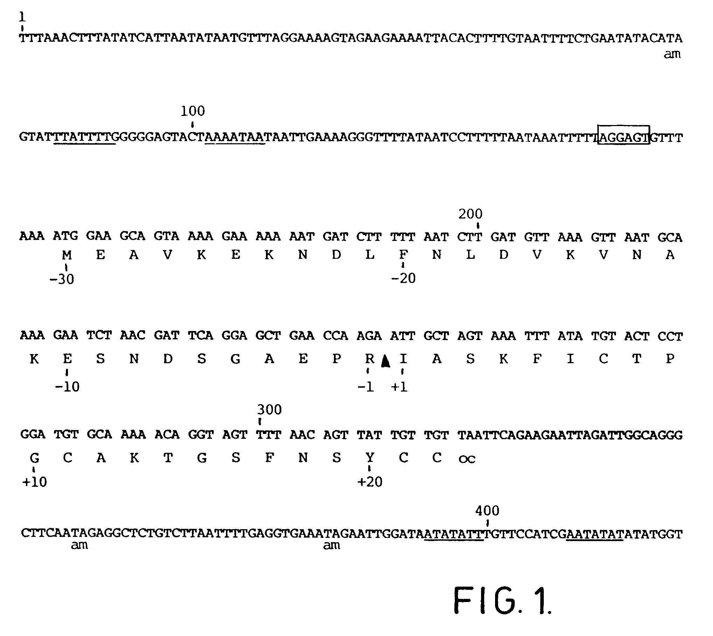

Fig. 1 is the nucleotide sequence of the epidermin structural gene (epi A) and the deduced amino-acid sequence of pre-epidermin. The Shine-Dalgarno sequence 2 base pairs in form of the ATG codon is boxed, and the proteolytic cleavage site at which the propeptide is processed is indicated by an arrow. Inverted repeats are underlined and potential stop codons are given (am amber; oc ochre).

The structural gene of pre-epidermin terminates at the TAA stop codon, hence pre-epidermin consist of 52 amino acids and it is processed to the epidermin between Arg⁻¹ and Ile⁺¹. Thus, as can clearly be seen, pre-epidermin is not a degredation product of a larger protein but is coded by a distinct structural gene.

Thus it is apparent that, unexpectedly, the precursor protein of the antibiotics are coded by distinct structural genes.

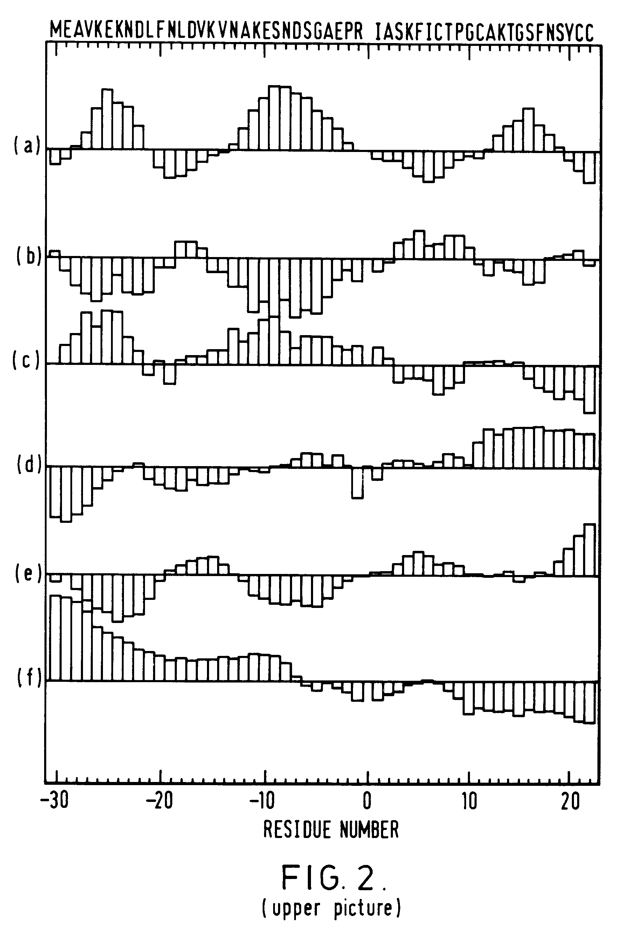

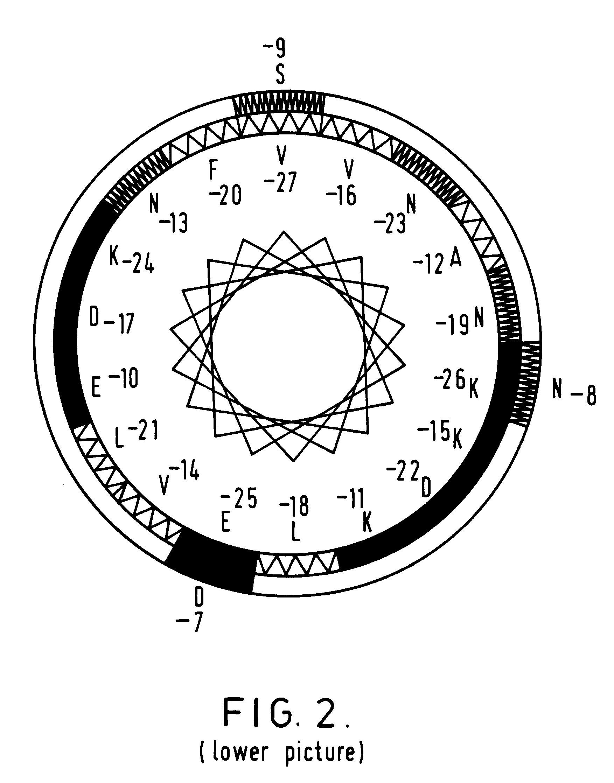

A combination of prediction profiles for secondary structure (α, β, turns), flexibility, hydropathy, hydrophilicity and helix wheel plot were made using a Hycon program and are shown in Fig. 2 (upper and lower pictures).

Fig. 2 (upper picture) is a prediction plot for pre-epidermin in which the respective bar charts show a) flexibility, b) hydropathy, d) propensities for turn, e) β-sheet and f) α-helix conformation.

Fig. 2 (lower picture) is the helix wheel plot which shows the N-terminus may partially adopt an amphiphilic α-helical conformation in an appropriate environment, possibly by interaction with the C-terminal pro-epidermin part which becomes highly lipophilic during sulphide ring formation. Modification steps occur at Ser and Thr residues in exposed turn structures.

A high α-helix probability is predicted for pre-epidermin -30 to -8 whereas the C-terminal part 1-22 which corresponds to pre-epidermin exhibits very high turn probability. Moreover, the prediction plot shows clearly, that the N-terminus -30 to -1 is highly hydrophilic, whereas the C-terminal part is more lipophilic. The N-terminal part -30 to -8 seems to fold partially into an amphiphilic α-helix.

The N-terminal segment of pre-epidermin -30 to -1 does not contain any cysteine residues, whereas the C-terminal segment 1-22 contains the four cystein residues, involved in sulphide bridge formation. Sequence -30 to -1 included many cleavage sites for endoproteases whereas even in the pre-epidermin state, sequence 1-22 is highly resistant to proteolytic degradation.

The mature antibiotic can only be attacked by trypsin at Lys in position 13. The processing site Arg⁻¹-Ile⁺¹ is hydrophilic and accessible, due to the turn forming Pro⁻² residue.

The various enzymatic reactions which occur in the production of the antibiotics such as epidermin include modifications of the pro-polypeptide part 1-22; cleavage of the N-terminal prepeptide fragment -30 to -1 and secretion of the matured antibiotic.

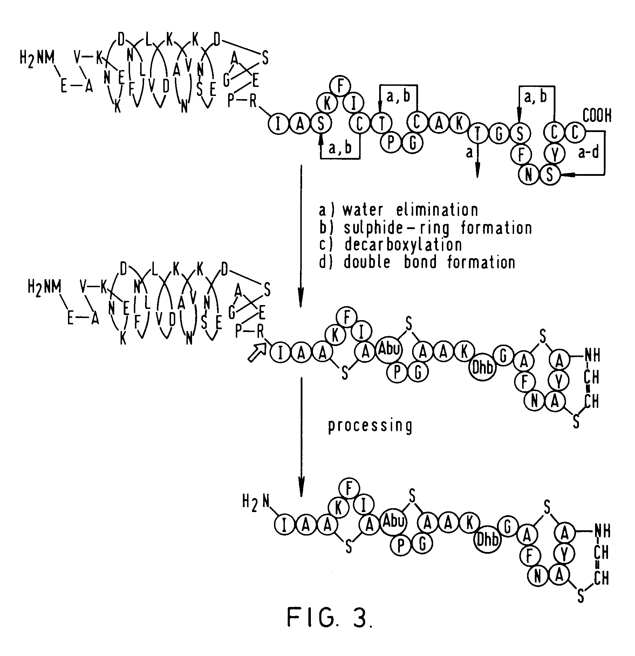

Fig. 3 illustrates the postulated maturation procedure of epidermin. The translated polypeptide (pre-epidermin) consists of 52-amino-acid residues. Structure predictions indicate a partially α-helical N-terminus from which residues -30 to -10 may form an amphiphilic α-helix conformation.

A Pro⁻² turn followed by an arginine residue corresponds to the processing site (a). A water elimination at Ser and Thr residues. With the exception of Thr⁺¹⁴, water elimination is followed by (b), sulphide ring formation, (c) describes the decarboxylation reaction, and (d) the double bond formation at the C-terminus. After these modifications, the pro-epidermin is proposed as an intermediate formation, from which the lantibiotic is processed (Abu, aminobutyrine acid; Dhb, dehydrobutryrine).

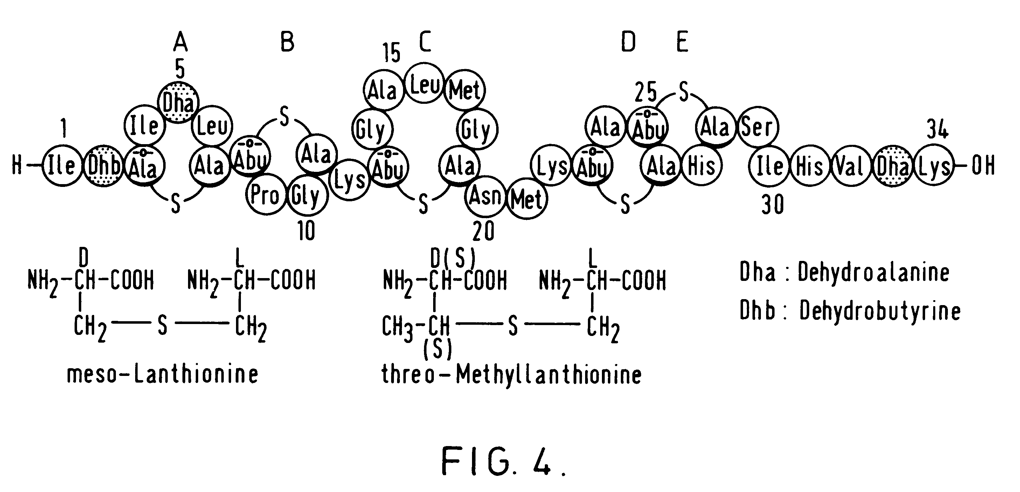

Fig. 4 illustrates the mature antibiotic epidermin. The various ring structures are designated A, B, C, D and E. The amino acids meso-lanthionine and threo-methyllanthionine, from which the various ring structures can be considered to be derived are shown.

The enzymatic modifications occur before cleavage of the prepeptide fragment. Enzymatic modification includes the elimination of water from Ser and Thr residues in position 5, 16, 19 and 8, 14 respectively to form dehydroalanine and dehydrobutyrine residues. Addition of thiol groups of Cys residues in position 2, 11, 21 and 22 to the C=C double bonds, also occurs, yielding the meso-lanthionine or (2S, 3S, 6R)-3-methyl-lanthionine bridges. In addition, decarboxylation of residue 22 and double bond formation yields the C-terminal S-(2-aminovinyl)-D-cysteine. The reaction of C-terminally situated cysteine thiol groups with N-terminally located dehydroamino acids occurs with complete stereo-specificity in epidermin, nisin and subtilin. Accordingly, during modification these elimination-addition reaction imply a reversal of configuration of the Cα carbon atoms at pre-epidermin residues. L-Ser and L-Thr to give D-configurated Cα atoms. On the other hand, the L-configuration of the cysteine halves is still maintained.

The four sulphide rings are also formed, subsequently at the same catalytic site, which is supported by the interaction with the N-terminal amphiphilic α-helix. Only Thr⁺¹⁴ dehydrates without finding a cysteine. This position (Lys⁺¹³-Dhb⁺¹⁴) constitutes the enzymatic cleavage site at which trypsin inactivates the antibiotic epidermin. During sulphide ring formation C-terminal rigidity and hydrophobicity increases and may favour interaction of pro-epidermin with the lipid bilayer and may induce translocation.

Finally, the hydrophilic α-helical N-terminus -30 to -1 is cleaved by a specific protease at the characteristic cleavage site described above.

Using the techniques described above plasmids coding for lantibiotics can be modified either by mutation of the gene coding for the respective polypeptide or by replacement of such a gene by a gene coding for a different polypeptide and used to transform the original host or a different host, provided such host also, in its native state, is capable of expressing a lantibiotic.

Generally speaking, where the original functional gene codes for a pre-sequence, as discussed above for example in the case of epidermin, the DNA sequence coding for such a pre-sequence may be retained in the modified plasmid; in this case the DNA-sequence for the new, or mutated pro-polypeptide will be positioned directly upstream of the pre-sequence DNA similarly to the original pro-polypeptide sequence.

Cultivation of a bacterial host according to the present invention may be carried out under conventionally used cultivation conditions as described for instance in our co-pending European Patent Application No. 89 108 350.3 and in European Patent Application Publication No. 0.181.578. Purification and isolation of the desired protein may also be carried out using the techniques or suitable modifications thereof described in the foregoing patent applications for epidermin and gallidermin, including the use of adsorbents, ion-exchange resins and if desired, HPLC.

The process of the invention can be applied to the formation of novel compounds for experimental purposes, or to the formation of known compounds or derivatives of known compounds in new hosts. For instance a plasmid containing the gene coding for epidermin can be used to transform the species Streptococcus lactis to produce epidermin from that host, or the gene coding for Gallidermin (see our co-pending European Patent Application referred to above) can be used to replace the gene coding for the pro-polypeptide for epidermin in e.g. plasmid pEpi32 and used to transform Staphylococcus epidermis DSM 3095 to produce gallidermin from this host. Similarly other biologically active peptide derivatives containing dehydroamino acid residues and/or lanthionine bridges and/or methyllanthionine bridges can be produced, such as derivatives of hormones such as human insulin, oxytosin, vasopresin, peptide antbiotics, hormone inhibitors such as elastase inhibitor and fibrinolytically active agents such as human tissue plasminogen activator. Such derivatives, as well as retaining biological activity of the parent compound can have increased stability and improved half-lives.

Ideally the DNA coding for the desired pro-polypeptide should include codons for cysteine and serine and/or for cysteine and threonine for the formation of thioether bridges.

For relatively short chain polypeptides these respective codons should normally be no more that eight and preferably no more than six codons apart, inclusive, although it is envisaged that, depending upon the steric conformation of the final polypeptide molecule much greater spacing is possible.

In respect of the formation of dehydroamino acids these will usually be derived from serine and threonine and, accordingly the DNA coding for the desired pro-polypeptide will include codons for such amino acids.

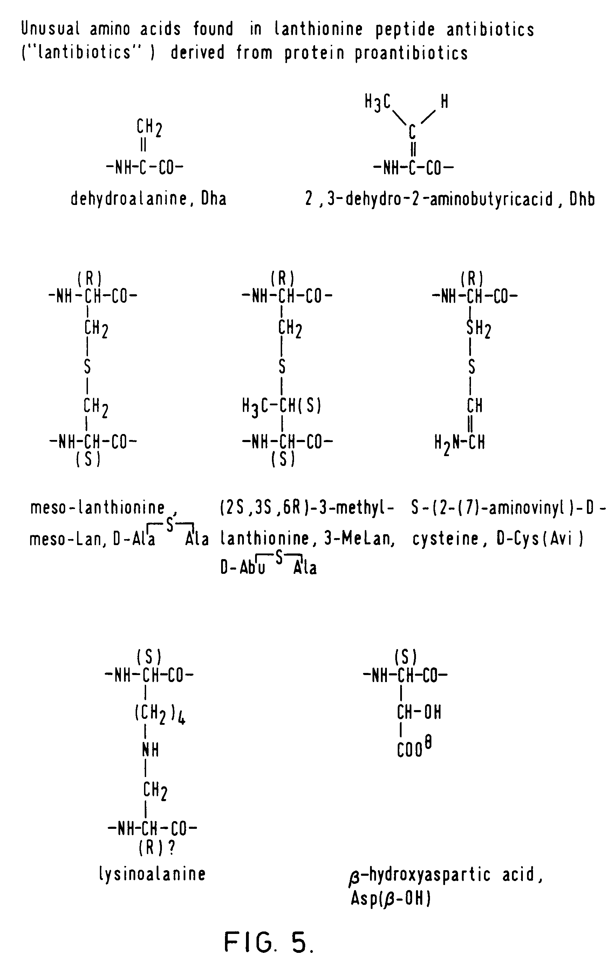

Amongst the unusual amino acids which may be present in a polypeptide produced according to the present invention are, dehydroalanine, 2,3-dehydro-2-aminobutyric acid, meso-lanthionine, (2S, 3S, 6R)-3-methyl-lanthionine, S-(2-(Z)-aminovinyl)-D-cysteine, lysinoalanine and β-hydroxyaspartic acid, the structure of these residues are shown in Fig. 5, which illustrated various unusual amino acids which are found in lanthionine antibiotics and which can be formed in peptide products by the practice of the present invention.

### Example 1

### 1. Overproduction of gallidermin

A DNA fragment containing the open reading frame of gallidermin can be cloned in Staphylococcus epidermidis DSM 3095, the epidermin producing strain by using a medium copy plasmid such as pC194, pE194, pUB110, pT181 or pMK148 gallidermin. An increase of the gene dosis usually correlates with an increase of product production; the correlation is not necessarily linear. High copy number plasmid derivatives of pC194 or pT181 can be used as cloning vehicles too.

### 2. Exchange of leader sequence

The leader-sequence of epidermin corresponding to amino acids -1 to -30, is involved in the secretion of epidermin. The sequence can be used to secrete other peptides in S. epidermidis such as gallidermin.

The leader-sequence DNA can be made portable by inserting respective linkers at the beginning and at the end of its sequence. Thus the leader sequence DNA can be isolated in large amounts from the plasmid and can be inserted at respective positions of other peptides and proteins. The leader-sequence DNA can also be produced by chemical synthesis.

### Example 2

### Production of Gallidermin using S.epidermis as host

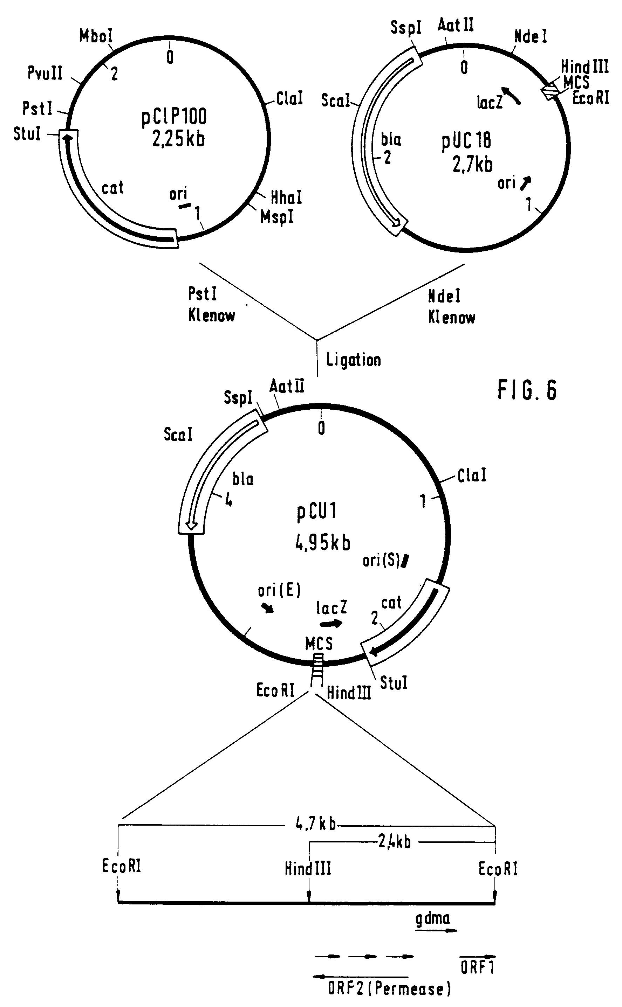

1. Preparation of plasmid (see Fig. 6)
   a) Plasmid pCUI was prepared by ligating Pstl digested pCLP100 and Ndel digested pUC18 using Klenow as described in the thesis "Molekular genetische Untersuchungen zur plasmidkodierten Arsenit und Arsenatrestistent bei Staphylococcen", Ralf Rosenstein, which was made available in the library of the Technischen Universität, München in 1988.
      The resulting plasmid was then digested with EcoRI.
   b) Chromosomal DNA was isolated from S.gallinarum (DMS 4616) and was digested with EcoRl. A 4.7 kb fragment containing the gallidermin structural gene in a 2.4 kb long sequence between HindIII and EcoRl restriction sites was isolated using as a primer the sequence
      5′ CAC ATC CAG GAG TAC 3′
   c) The 4.7 kb Fragment was then ligated into the EcoRl site of the digested pCUI pasmid from step a) to give a plasmid designated pCUgdml.
2. Preparation of a S.epidermis host
   In this example a mutant strain of S.epidermis DSM 3095 incapable of producing epidermin was isolated.
   The mutagenis was carried out on a strain which was characterised by chromosomally coded Rifampicin resistance (20 ug/ml).
   S.epidermis DSM 3095 grown on Agar plates was used to inoculate 30 ml basic broth medium which was cultivated over night. 0.5 ml of the overnight cultivation was then used to inoculate 50 ml of production medium which was shake cultivated at 37°C for three hours.
   Cells were removed from the cultivation medium and suspended in 4.5 ml pre-warmed TM-Buffer (30 mM Tris-Maleate pH 6.5 (the resulting solution is designated Solution A).
   The solution A was checked for spontaneous mutations and for cell count (1.25 x 10¹⁰ cells/ml).
   4 ml of solution A was thoroughly shaken with 1 ml ethyl methyl sulphonate (final concentration 47 g/ml) and then maintained under shaking at 37°C for one hour.
   Cells were then extracted from the cultivation broth, washed twice in TM-Buffer and resuspended in 5 ml TM-Buffer (the resulting solution was designated Solution B and contained mutated cells).
   Solution B was found to contain 2 x 10⁸ cells/ml which corresponds to a survival rate of 1.6%.
   50 l of solution B was added to 5 ml production medium and grown overnight at 37°C (phenotypic expression). The resulting solution was designated Solution C. A cell count showed 7.3 x 10⁸ cells/ml.
   The solution was plated on BM-Agar plates and individual colonies were picked out. These were used to inoculate test plates (consisting of BM-Agar to which Micrococcus luteus has been laid on the surface). Those colonies which had no inhibitory effect on M. luteus were selected as non-producers of Epidermin.
   BM Agar contains per litre
   10 gm Peptone No. 140
   5 gm Yeast extract
   1 mg Glucose
   5 mg NaCl
   1 mg K₂HPO₄
   pH 7.5
   A mutation rate of about 3% was noted.
   The 45 non-producers which were found were sub-cloned 20 times to yield 16 stable non-producers.
   All stable non-producers were found to contain the wild type plasmid pEpi32. From the restriction pattern this is identified as identical to the plasmid in the wild type strain.

### Transformation of non-producing S.epidermis

750 ml of BM-medium was inoculated with 5 ml of medium obtained by overnight cultivation of a stable non-producing strain, and the inocculated medium was shake cultivated in a 2 litre flask at 37°C with a shake speed of 120 rpm.

The initial optical density of the inocculated BM-medium was 0.03-0.04. When the optical density had reached 0.45-0.55 the cells were removed by centrifugation in a GS.-3-Rotor at 8500 rpm for 15 minutes at 4°C. The isolated cells were then washed successively in 750, 350, 40 and 10 ml of 10% glycerin, suspended in 2-3 ml 10% glycerin, and frozen in 110 l portions in ERGs at -70°C. The cell count amounted to 1-5 x 10¹⁰/ml
The frozen cells were thawed at room temperature for 5 minutes, then 50 l of cell suspension was incubated in an ERG with 2 l plasmid pCUgdml in TE-Buffer for 30 minutes at room temperature.

The mixture was then introduced into an electroporation curvette having a 0.2 cm electrode gap and immediately electroporated. Thereafter the cells were rapidly resuspended in 950 l SMMP50-medium, transferred into a
2.5 ml ERG and shaken for 90 minutes at 37°C. The ERGs were inclined at 45^{o} in order to provide for a good aeration of the medium.

SMMP50-medium contains pro 100 ml, 55 ml 2SMM, 40 ml 4 PAB and 5 mol 5 % BSA. The 2SMM contains 1 mol saccharose, 0.04 mol maleic acid, 0,04 mol MgCl₂ and NaOH to pH 6.5. 4 PAB is a solution of 7 g/100 ml of Gibco antibiotic medium 3.

The cell suspension is diluted and spread on a BM-Agar containing gallidermin which is incubated for 20 hours at 37°C.

Testing of growing strains which produce gallidermin was carried out by selection of colonies from a M. luteus test plate and by cultivating the respective selected colonies and determining the presence of gallidermin by HPLC.

Three pCUgdml transformed mutants capable of producing gallidermin were located.

### Determination of the presence of gallidermin produced by pCUgdml transformed S.epidermin

a) Bio assay
FP-Agar was inocculated with M. luteus ATCC 9341 and incubated at 37°C for 18 hours. Half of the produced culture was removed with a loop and suspended in 100 ml FP-medium and was cultivated for 8 hours at 36°C. The cultivation was stopped when the optical density reached 1.0. FP-Agar was inoculated with 0.5% of this suspension, each 10 ml was poured into a Petri dish and stored for 3 weeks at 4°C.
The Plate diffusion test was carried out as described in Zähner and Maas, "Biology of Antibiotics" Springer Verlag, Berlin 1972. 10 ul of culture filtrate from cultivation of the transformed S.epidermin was captured on a filter paper and dried. The paper was placed on the test plate which was then incubated for 24 hours at 37°C.
b) HPLC
The selected transformed strain was cultivated for 26 hours in the production medium. The culture broth was centrifuged for 10 minutes at 13.000 rpm.
The isolated culture liquid was then subject to HPLC on a SP 8.700 liquid chromatography apparatus (Spectra Physics, Darmstadt, FRG) using as the mobile phase A) H₂O with 0.5% 70% perchloric acid and B) Acetonitril. Column packings were Nucleosil -100 C-18 of grain size 7 um and column sizes 125 mm x 4.6 mm I.D. and 20 mm x 4.6 mm ID for the pre-column.
Gradients were as follows:

| time (min.) | A [%] | B [%] |
|---|---|---|
| 0 | 77.5 | 22.5 |
| 8 | 63.0 | 37.0 |
| 8.5 | 0 | 100 |
| 9.5 | 0 | 100 |
| 10 | 77.5 | 22.5 |
| 14 | 77.5 | 22.5 |

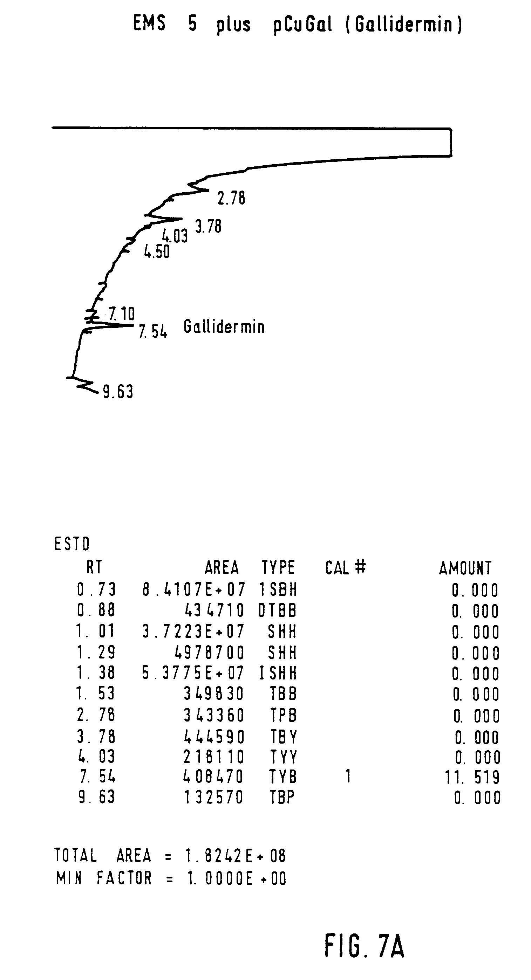

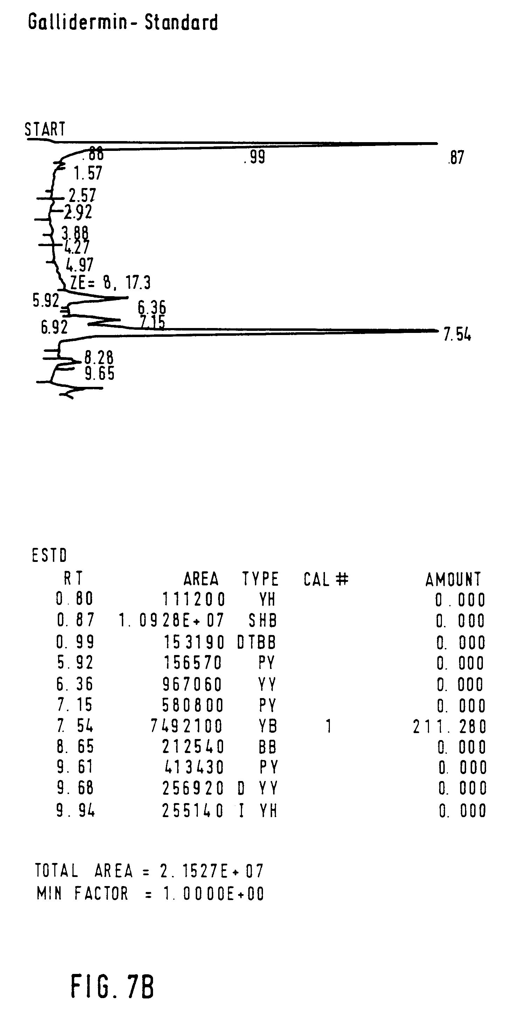

The resulting chromatogram is shown in Fig. 7A. A standard curve is shown in Fig. 7B showing that gallidermin elutes at 7.54 minutes.

The following were used as culture medium

| 1. FP-Agar | |
|---|---|
| Meat extract | 4 g |
| Peptone | 10 g |
| NaCl | 3 g |
| Na₂HPO₄ | 5 g |
| Glucose | 10 g |
| Complex agar | 15 g |
| Water | 1 litre |
| pH | 7.2 |

| 2. FP-Medium | |
|---|---|
| Meat extract | 4 g |
| Peptone | 10 g |
| NaCl | 3 g |
| Na₂HPO₄ | 5 g |
| Glucose | 10 g |
| Water | 1 litre |
| pH | 7.2 |

| 3. Production medium | |
|---|---|
| Meat extract | 33 g |
| Malt extract | 30 g |
| NaCl | 40 g |
| Calcium Hydroxide | 3.8 g |
| Water | 1 litre |
| pH | 6.5 |

## Claims

1. A method of preparing a plasmid which comprises ligating together i) a first DNA molecule including a sequence coding for a multi-enzyme complex which can effect water elimination and sulphide bridge formation and optionally decarboxylation and double bond formation of a precursor polypeptide and ii) a second DNA molecule which includes a gene coding for a said precursor polypeptide.

2. A method according to claim 1, wherein the first DNA molecule comprises a plasmid from which a gene coding for a precursor polypeptide has been excised and the gene contained in the second DNA molecule codes for a precursor polypeptide different from that coded for by the gene which has been excised from the plasmid.

3. A method according to claim 1 or 2, wherein the first DNA molecule is derived from the plasmid pEpi32 (DSM 3095) and the second DNA molecule includes a gene coding for an antibiotic other than epidermin, or a hormone or a fibrinolytic agent.

4. A method according to any one of claims 1 to 3, in which the resulting plasmid contains a nucleotide sequence coding for the pre-peptide sequence -30 to -1 of pre-epidermin and for a pro-peptide sequence which is different from epidermin.

5. A method according to claim 5, in which the pro-peptide sequence codes for gallidermin.

6. A method according to claim 4, in which the second DNA sequence includes a gene coding for gallidermin.

7. A recombinant DNA molecule which comprises a plasmid containing i) a first DNA sequence which codes for a multi-enzyme complex which can effect water elimination and sulphide bridge formation and optionally decarboxylation and double bond formation of a precursor polypeptide, and which, in a plasmid in its natural host, is capable of expressing said multi-enzyme complex, and ii) a second DNA sequence which contains a gene coding for a precursor polypeptide; and in which the second DNA sequence is foreign to the plasmid.

8. A recombinant DNA molecule wherein the gene contained in the second DNA sequence codes for an antibiotic, a hormone or a fibrinolytic agent.

9. A recombinant DNA molecule according to claim 7 or claim 8, wherein the first DNA sequence is derived from the plasmid pEpi32 (DSM 3095) and the second DNA molecule includes a gene coding for an antibiotic other than epidermin, or a hormone or a fibrinolytic agent.

10. A recombinant DNA molecule according to any one of claims 7 to 9, containing a nucleotide sequence coding for the pre-peptide sequence -30 to -1 of pre-epidermin and for a pro-peptide sequence which is different from epidermin.

11. A recombinant DNA molecule according to claim 10, wherein the pro-peptide sequence codes for gallidermin.

12. A recombinant DNA molecule according to claim 9, in which the second DNA sequence includes a gene which codes for gallidermin.

13. A bacterial host containing a plasmid, wherein said plasmid codes for a polypeptide which is not normally produced by said host, and wherein said host during cultivation provides a multi-enzyme complex capable of effecting water elimination, and/or sulfide bridge formation, and possibly also decarboxylation and double bond formation, whereby a peptide is produced which contains at least one dehydroamino acid and/or at least one lanthionine bridge, said produced polypeptide being foreign to said host; but excluding a bacterial host which is a bacterium of the genus Streptococcus containing a plasmid which codes for Nisin production.

14. A bacterial host according to claim 13, which is Streptococcus lactis, Bacillus subtilis, Streptomyces cinnamoneous, Streptomyces sp. Streptoverticullum griseoverticillum, Staphylococcus epidermis, Staphylococcus epidermin strain 5 or Staphylococcus gallinarum.

15. A bacterial host according to claim 1 or claim 2, wherein the plasmid codes for an antibiotic, a hormone or a fibrinolytic agent.

16. A bacterial host according to any one of claims 1 to 3, in which the host is staphylococcus epidermis DSM 3095 or a functionally equivalent mutant thereof and the plasmid contains a gene coding for gallidermin.

17. A bacterial host containing a plasmid as defined in any one of claims 7 to 12.

18. A process which comprises cultivating a bacterial host according to any one of claims 13 to 17 and isolating a resulting peptide product containing at least one dehydroamino acid residue and/or one lanthionine bridge.

## Patentansprüche

1. Verfahren zur Herstellung eines Plasmids, bei welchem Verfahren zusammenligiert werden: i) ein erstes DNA-Molekül, das eine Sequenz enthält, die für einen Multi-Enzym-Komplex codiert, der Wassereliminierung und Sulfidbrückenbildung sowie gegebenenfalls Decarboxylierung und Doppelbindungsbildung eines Precursor-Polypeptids bewirken kann, und ii) ein zweites DNA-Molekül, das ein Gen enthält, das für ein genanntes Precursor-Polypeptid codiert.

2. Verfahren nach Anspruch 1, bei welchem das erste DNA-Molekül ein Plasmid umfaßt, aus welchem ein für ein Precursor-Polypeptid codierendes Gen herausgeschnitten wurde, und das in dem zweiten DNA-Molekül enthaltene Gen für ein Precursor-Polypeptid codiert, das sich von jenem unterscheidet, für das das aus dem Plasmid herausgeschnittene Gen codiert.

3. Verfahren nach Anspruch 1 oder 2, bei welchem das erste DNA-Molekül von dem Plasmid pEpi32 (DSM 3095) stammt und das zweite DNA-Molekül ein Gen umfaßt, das für ein von Epidermin unterschiedliches Antibiotikum oder ein Hormon oder ein fibrinolytisches Agens codiert.

4. Verfahren nach irgendeinem der Ansprüche 1 bis 3, bei welchem das resultierende Plasmid eine Nucleotidsequenz enthält, die für die Pre-Peptid-Sequenz -30 bis -1 von Pre-Epidermin und für eine Pro-Peptid-Sequenz, die sich von Epidermin unterscheidet, codiert.

5. Verfahren nach Anspruch 5, bei welchem die Pro-Peptid-Sequenz für Gallidermin codiert.

6. Verfahren nach Anspruch 4, bei welchem die zweite DNA-Sequenz ein für Gallidermin codierendes Gen enthält.

7. Rekombinantes DNA-Molekül, umfassend ein Plasmid, das enthält: i) eine erste DNA-Sequenz, die für einen Multi-Enzym-Komplex codiert, der Wassereliminierung und Sulfidbrückenbildung sowie gegebenenfalls Decarboxylierung und Doppelbindungsbildung eines Precursor-Polypeptids bewirken kann, und die in einem Plasmid in dessen natürlichem Wirt zur Expression dieses Multi-Enzym-Komplexes befähigt ist, sowie ii) eine zweite DNA-Sequenz, die ein für ein Precursor-Polypeptid codierendes Gen enthält; wobei die zweite DNA-Sequenz für das Plasmid fremd ist.

8. Rekombinantes DNA-Molekül, in welchem das in der zweiten DNA-Sequenz enthaltene Gen für ein Antibiotikum, ein Hormon oder ein fibrinolytisches Agens codiert.

9. Rekombinantes DNA-Molekül nach Anspruch 7 oder 8, in welchem die erste DNA-Sequenz von dem Plasmid pEpi32 (DSM 3095) stammt und das zweite DNA-Molekül ein Gen enthält, das für ein anderes Antibiotikum als Epidermin, für ein Hormon oder ein fibrinolytisches Agens codiert.

10. Rekombinantes DNA-Molekül nach irgendeinem der Ansprüche 7 bis 9, das eine Nucleotidsequenz enthält, die für die Pre-Petid-Sequenz -30 bis -1 von Pre-Epidermin und für eine Pro-Peptid-Sequenz, die sich von Epidermin unterscheidet, codiert.

11. Rekombinantes DNA-Molekül nach Anspruch 10, in welchem die Pro-Peptid-Sequenz für Gallidermin codiert.

12. Rekombinantes DNA-Molekül nach Anspruch 9, in welchem die zweite DNA-Sequenz ein Gen enthält, das für Gallidermin codiert.

13. Bakterieller Wirt, der ein Plasmid enthält, welches für ein Polypeptid codiert, das normalerweise von diesem Wirt nicht produziert wird, wobei dieser Wirt während der Züchtung einen Multi-Enzym-Komplex bildet, der zur Wassereliminierung und Sulfidbrückenbildung sowie gegebenenfalls zur Decarboxylierung und Doppelbindungsbildung befähigt ist, wobei weiters ein Peptid produziert wird, das zumindest eine Dehydroaminosäure und/oder zumindest eine Lanthioninbrücke enthält, und das produzierte Polypeptid für den genannten Wirt fremd ist; wobei jedoch ein bakterieller Wirt ausgeschlossen ist, der ein Bakterium der Gattung Streptococcus ist, das ein für Nisin-Produktion codierendes Plasmid enthält.

14. Bakterieller Wirt nach Anspruch 13, der Streptococcus lactis, Bacillus subtilis, Streptomyces cinnamoneus, Streptomyces sp. Streptoverticullum griseoverticillum, Staphylococcus epidermis, Staphylococcus epidermin Stamm 5 oder Staphylococcus gallinarum ist.

15. Bakterieller Wirt nach Anspruch 1 oder 2, in welchem das Plasmid für ein Antibiotikum, ein Hormon oder ein fibrinolytisches Agens codiert.

16. Bakterieller Wirt nach irgendeinem der Ansprüche 1 bis 3, in welchem der Wirt Staphylococcus epidermis DSM 3095 oder eine funktionell äquivalente Mutante davon ist und das Plasmid ein für Gallidermin codierendes Gen enthält.

17. Bakterieller Wirt, der ein Plasmid nach der Definition von irgendeinem der Ansprüche 7 bis 12 enthält.

18. Verfahren, umfassend die Züchtung eines bakteriellen Wirts nach irgendeinem der Ansprüche 13 bis 17 und die Isolierung eines entstehenden Peptidprodukts, das zumindest einen Dehydroaminosäurerest und/oder eine Lanthioninbrücke enthält.

## Revendications

1. Procédé de préparation d'un plasmide qui comprend la ligature l'une à l'autre i) d'une première molécule d'ADN contenant une séquence codant un complexe multi-enzyme qui peut réaliser une élimination d'eau et la formation de ponts sulfure et éventuellement une décarboxylation et la formation de doubles liaisons d'un polypeptide précurseur et ii) une seconde molécule d'ADN qui contient un gène codant ledit polypeptide précurseur.

2. Procédé selon la revendication 1, dans lequel la première molécule d'ADN comprend un plasmide duquel un gène codant un polypeptide précurseur a été excisé et le gène contenu dans la seconde molécule d'ADN code un polypeptide précurseur différent de celui qui est codé par le gène qui a été excisé du plasmide.

3. Procédé selon la revendication 1 ou 2, dans lequel la première molécule d'ADN est dérivée du plasmide pEpi32 (DSM 3095) et la seconde molécule d'ADN contient un gène codant un antibiotique différent de l'épidermine, ou une hormone ou un agent fibrinolytique.

4. Procédé selon l'une quelconque des revendications 1 à 3, dans lequel le plasmide résultant contient une séquence nucléotidique codant la séquence prépeptidique -30 à -1 de la pré-épidermine et une séquence propeptidique qui est différente de l'épidermine.

5. Procédé selon la revendication 4 dans lequel la séquence propeptidique code la gallidermine.

6. Procédé selon la revendication 4 dans lequel la seconde séquence d'ADN contient un gène codant la gallidermine.

7. Molécule d'ADN recombinant qui comprend un plasmide contenant i) une première séquence d'ADN qui code un complexe multi-enzyme qui peut réaliser une élimination d'eau et la formation de ponts sulfure et éventuellement une décarboxylation et la formation de doubles liaisons d'un polypeptide précurseur, et qui est capable, dans un plasmide dans son hôte naturel, d'exprimer ledit complexe multienzyme, et ii) une seconde séquence d'ADN qui contient un gène codant un polypeptide précurseur, et dans lequel la seconde séquence d'ADN est étrangère au plasmide.

8. Molécule d'ADN recombinant dans laquelle le gène contenu dans la seconde séquence d'ADN code un antibiotique, une hormone ou un agent fibrinolytique.

9. Molécule d'ADN recombinant selon la revendication 7 ou la revendication 8, dans laquelle la première séquence d'ADN est dérivée du plasmide pEpi32 (DSM 3095) et la seconde séquence d'ADN contient un gène codant un antibiotique différent de l'épidermine, ou une hormone ou un agent fibrinolytique.

10. Molécule d'ADN recombinant selon l'une quelconque des revendications 7 à 9, contenant une séquence nucléotidique codant la séquence prépeptidique -30 à -1 de la pré-épidermine et une séquence propeptidique qui est différente de l'épidermine.

11. Molécule d'ADN recombinant selon la revendication 10, dans laquelle la séquence propeptidique code la gallidermine.

12. Molécule d'ADN recombinant selon la revendication 9, dans laquelle la seconde séquence d'ADN contient un gène qui code la gallidermine.

13. Hôte bactérien contenant un plasmide, dans lequel ledit plasmide code un polypeptide qui n'est pas produit normalement par ledit hôte, et dans lequel ledit hôte fournit en cours de culture un complexe multi-enzyme capable de réaliser une élimination d'eau et/ou la formation de ponts sulfure, et éventuellement aussi une décarboxylation et la formation de doubles liaisons, de sorte qu'il y a production d'un peptide qui contient au moins un déshydroaminoacide et/ou au moins un pont lanthionine, ledit polypeptide produit étant étranger audit hôte, mais excluant un hôte bactérien qui est une bactérie du genre Streptococcus contenant un plasmide qui code la production de nisine.

14. Hôte bactérien selon la revendication 13, qui est Streptococcus lactis, Bacillus subtilis, Streptomyces cinnamoneus, Streptomyces sp. Streptoverticullum griseoverticillum, Staphylococcus epidermis, Staphylococcus epidermin souche 5 ou Staphylococcus gallinarum.

15. Hôte bactérien selon la revendication 1 ou la revendication 2, dans lequel le plasmide code un antibiotique, une hormone ou un agent fibrinolytique.

16. Hôte bactérien selon l'une quelconque des revendications 1 à 3, dans lequel l'hôte est Staphylococcus epidermis DSM 3095 ou un mutant fonctionnellement équivalent de celui-ci et le plasmide contient un gène codant la gallidermine.

17. Hôte bactérien contenant un plasmide tel que défini dans l'une quelconque des revendications 7 à 12.

18. Procédé qui comprend la culture d'un hôte bactérien selon l'une quelconque des revendications 13 à 17 et l'isolement d'un produit peptidique résultant contenant au moins un résidu de déshydroaminoacide et/ou un pont lanthionine.
